Europäisches Patentamt

European Patent Office

Office européen des brevets

(11) Veröffentlichungsnummer : **0 174 519**
**B1**

(12) # EUROPÄISCHE PATENTSCHRIFT

(45) Veröffentlichungstag der Patentschrift :
20.01.88

(21) Anmeldenummer : 85110360.6

(22) Anmeldetag : 19.08.85

(51) Int. Cl.⁴ : **C 07 C143/66**

(54) **Verfahren zur Herstellung von 1-Amino-2-naphthol-4-sulfonsäure.**

(30) Priorität : 29.08.84 DE 3431695

(43) Veröffentlichungstag der Anmeldung :
19.03.86 Patentblatt 86/12

(45) Bekanntmachung des Hinweises auf die Patenterteilung : 20.01.88 Patentblatt 88/03

(84) Benannte Vertragsstaaten :
CH DE FR GB LI

(56) Entgegenhaltungen :
EP-A- 0 080 644
ULLMANNS ENCYKLOPÄDIE DER TECHNISCHEN
CHEMIE, 4. Auflage, Band 17, 1974, VERLAG CHEMIE,
Weinheim, New York

(73) Patentinhaber : **BAYER AG**
**Konzernverwaltung RP Patentabteilung**
**D-5090 Leverkusen 1 Bayerwerk (DE)**

(72) Erfinder : **Linhart, Karl, Dr.**
**Heymannstrasse 5**
**D-5090 Leverkusen 1 (DE)**

## Beschreibung

1-Amino-2-naphthol-4-sulfonsäure (Amidolsäure) ist ein wichtiges Zwischenprodukt zur Herstellung von 6-Nitro-1-diazo-2-naphthol-4-sulfonsäure einer Azokomponente zur Herstellung von Chromkomplexfarbstoffen. Die Herstellung von 1-Amino-2-naphthol-4-sulfonsäure ist in Organic Synthesis, Col. Vol. I 1941, S. 411 und Vol. II, 1943, S. 42 beschrieben. Gemäß dieser Vorschrift wird die Umsetzung mit Natriumnitritlösung in einer verdünnten wäßrigen Lösung durchgeführt und die isolierte Paste des nitrosierten β-Naphthols mit der 2,7-fachen molaren Menge Natriumhydrogensulfit umgesetzt. Anschließend wird mit Schwefelsäure angesäuert und das Produkt isoliert. Die Ausbeuteangaben beziehen sich auf die erhaltene Trockenmasse, deren Anteil an Amidolsäure ungenannt bleibt.

1-Amino-2-naphthol-4-sulfonsäure (Amidolsäure) wird auch gemäß BIOS Final Report N°. 986, Pt, 1 Item N°. 22, S. 44-45 und S. 146-147 in wäßriger Lösung hergestellt, wobei Ausbeuten von 80 % erhalten werden. Bei diesem Verfahren entsteht pro 1 Mol β-Naphthol 200 g Natriumsulfat, welches eine große Belastung für das Abwasser darstellt.

In der Europäischen Patentanmeldung Nr. 0080644 wird ein Verfahren zur Herstellung von 1-Amino-2-naphthol-4-sulfonsäure (Amidolsäure) durch Ansäuern von 3-Oxo-4-hydroxyimino-1,2,3,4-tetrahydronaphthalin-1-sulfonsäure oder ihres Alkalisalzes beschrieben. Die Autoren setzen isolierte und umkristallisierte 3-Oxo-4-hydroxyimino-1,2,3,4-tetrahydronaphthalin-1-sulfonsäure in Form ihres Natriumsalzes ein und reduzieren mit der 1,5-fachen molaren Menge Natriumhydrogensulfit in Gegenwart von 0,8 Mol Eisen-II-sulfat. Die Ausbeute liegt bei 90 % bezogen auf 3-Oxo-4-hydroxyimino-1,2,3,4-tetrahydronaphthalin-1-sulfonsäure.

Alle diese Verfahren haben den Nachteil, daß sie in sehr verdünnten Lösungen durchgeführt werden müssen und große Mengen Natriumhydrogensulfit erforderlich sind.

Um β-Naphthol in fein verteilbarer, reaktionsfähiger Form zu erhalten, muß dieses zunächst mit großen Mengen Natronlauge gelöst und dann wieder mit Schwefelsäure ausgefällt werden.

Hierbei entstehen größere Mengen Natriumsulfat. Neben der geringen Raum-Zeit-Ausbeute und dadurch bedingten hohen Investitionskosten führen die genannten Verfahren zu hohem Wasserverbrauch und vor allem zu hoher Abwasserbelastung.

Gegenstand der vorliegenden Erfindung ist nun ein Verfahren, bei dem β-Naphthol in Gegenwart von wassermischbaren ein- und mehrwertigen Alkoholen, deren Ethern oder Ester, cyclischer Ether, Sulfone, Amide, Nitrile, Lactame, Lactone, Sulfolan oder Sulfolen und deren in α- oder β-Stellung durch Alkyl oder Hydroxylalkyl substituierten Derivaten, Dimethylsulfoxid, Dioxan oder Glykolformal und einer Säure mit Nitriten nitrosiert und anschließend mit Hydrogensulfit direkt zu Salzen der Amidolsäure umgesetzt wird. Durch Zusatz von Säuren wird die Amidolsäure isoliert.

Geeignete derartige Lösungsmittel sind beispielsweise Ethylenglykol, Propylenglykol, Di- und Triethylenglykol, 2-Methylpentandiol-2,4, Ethylenglykolmonomethyl-, -ethyl- oder -butylether, Diethylenglykolmonoethyl, -ethyl oder -butylether, Diethylenglykolmonoethyl etheracetat, Triethylenglykolmonobutylether, Dipropylenglykol, Glycerin, Glycerin-2,4-diethylether, Thiodiglykol, Formamid oder N,N-Dimethylformamid, N,N-Dimethylacetamid, N,N-Dimethylmethoxyacetamid, N,N,N',N'-Tetramethylharnstoff, N-Methylpyrrolidon, 1,5-Dimethylpyrrolidon, γ-Butyrolacton, Acetonitril, β-Hydroxypropionitril, Ethyllacetat, Ethylencarbonat, Diethylenglykolmonoacetat, Diacetonalkohol oder Acetonylaceton, Isopropylalkohol, Tetrahydrofurfurylalkohol, (Glycerinformal) (5-Oxy-1,3-dioxan), Sulfolan (Tetramethylensulfon, Tetrahydrothiophen-S-dioxid) (Dioxan), Glykolformal (1,3-Dioxolan) oder deren Mischungen.

Bevorzugte Lösungsmittel sind $C_1$-$C_4$-Alkohole, wie Methanol, Ethanol, n-Propanol, Isopropanol oder n-Butanol und Isobutanol, Pyrrolidon und N-Methylpyrrolidon.

Die Mengen an organischen Lösungsmitteln können in weiten Grenzen variieren. Im allgemeinen haben sich Mengen von 5 bis 50 %, bezogen auf das gesamte Reaktionsgemisch, vorzugsweise 7 bis 20 % bewährt.

Als Nitrite zur Nitrosierung können Alkali- oder Erdalkalinitrite, sowie organische Nitrite, beispielsweise die in der DE-A 21 44 420 beschriebenen Glykolnitrite verwendet werden. Vorzugsweise wird Natriumnitrit eingesetzt.

Als Säuren können sowohl anorganische, als auch organische Säuren verwendet werden, beispielsweise Halogenwasserstoffsäuren, wie Salzsäure, Phosphorsäure, Phosphonsäuren, wie Methanphosphonsäure, phosphorige Säure, vorzugsweise Schwefelsäure. Als organische Säuren können wasserlösliche Carbonsäuren, wie Ameisen-, Essig-, Propion-, Monochloressig-, Dichloressig- und Trichloressigsäure, Milchsäure, Benzolsulfonsäure oder Toluolsulfonsäure, vorzugsweise Essigsäure und Propionsäure verwendet werden.

Als Säuren zum Isolieren der Amidolsäure können die obengenannten, vorzugsweise anorganischen Säuren, insbesondere Schwefelsäure verwendet werden.

Als Hydrogensulfite werden beispielsweise Alkali-, Erdalkali- oder Ammoniumhydrogensulfite eingesetzt, vorzugsweise Natriumhydrogensulfit.

Das Verfahren wird zweckmäßig in der Weise durchgeführt, daß man β-Naphthol in dem wassermischbaren organischen Lösungsmittel gegebenenfalls bei erhöhter Temperatur löst. Diese Lösung wird mit der äquimolekularen Menge (bezogen auf β-Naphthol) einer wäßrigen Lösung des Nitrits versetzt und die erhaltene Mischung bei etwa — 10 bis + 10 °C vorzugsweise bei 0 bis 5 °C mit mindestens der äquimolaren Menge einer Säure insbesondere einer Mineralsäure innerhalb von 1 bis 10 Stunden, vorzugsweise 2 bis 5 Stunden versetzt.

Nach beendeter Zugabe der Säure läßt man die Mischung gegebenenfalls bei erhöhter Temperatur 1 bis 5 Stunden nachreagieren. Danach wird die Reaktionsmischung mit geringen Mengen Alkali auf pH 5 bis 7 eingestellt und mit mindestens der 2-fachen molaren Menge (bezogen auf β-Naphthol) einer wäßrigen Alkalihydrogensulfitlösung versetzt. Nach einer Reaktionszeit von 8 bis 15 Stunden kann die Amidolsäure durch Zugabe einer starken Säure ausgefällt werden.

Die nach dem erfindungsgemäßen Verfahren erhaltenen Reaktionsmischungen enthalten etwa 10 bis 20 Gew.-% Amidolsäure, bezogen auf das Gewicht des Reaktionsgemisches vor der Ausfüllung mit Säure.

Zu gleich guten Ergebnissen gelangt man auch, wenn das β-Naphthol in einer Mischung aus dem organischen Lösungsmittel und einer organischen Säure, vorzugsweise Ameisen- oder Essigsäure, gelöst wird, diese Mischung mit Nitrit versetzt wird und anschließend ohne weitere Zugabe einer Säure wie oben weitergearbeitet wird.

Der Vorteil des Verfahrens liegt darin, daß die Amidolsäure in hohen Ausbeuten und hoher Reinheit erhalten wird, ohne daß große Mengen an überschüssigen Reagenzien, wie Säuren, Alkalien und Sulfiten, verwendet werden müssen.

Von besonderem Vorteil ist, daß das Verfahren in wesentlich konzentrierterer Form als bisher bekannte Verfahren durchgeführt wird. Neben einer höheren Raum-Zeit-Ausbeute und dadurch verringerten Investitionskosten führt das Verfahren zu einer wesentlich verringerten Abwasserbelastung.

Beispiel 1

144 g (1 Mol) β-Naphthol werden in 200 ml Isopropanol bei 40 °C gelöst. Dann läßt man 230 ml $NaNO_2$ 30 Vol.-%ig (1 Mol) zulaufen und kühlt auf 0 °C ab. Bei 0 bis 5 °C läßt man innerhalb von 4 Stunden 100 g $H_2SO_4$ 50 %ig zutropfen und rührt 60 Minuten ohne Kühlung nach. Anschließend wird mit ca. 15 ml NaOH 40 Vol.-%ig auf pH 6,0 eingestellt. Innerhalb von 20 Minuten läßt man dann 532 g $NaHSO_3$ 40 %ig (2,05 Mol) zulaufen. Nach Zugabe sollte der pH-Wert nicht unter 5,5 liegen, gegebenenfalls wird mit NaOH nachgestellt. Es wird solange bei Raumtemperatur gerührt, bis bei der polarographischen Kontrolle der Sulfit-Gehalt unter 0,1 % liegt. Dann gibt man in 2 Stunden 142,6 g $H_2SO_4$ 96 %ig zu. Die Temperatur steigt langsam an und sollte bei 60 °C gehalten werden. Nach ca. 5 bis 6 Stunden wird auf 10 °C abgekühlt und abgepreßt. Der Preßkuchen wird mit 500 ml $H_2O$ neutral gewaschen.

Pastengewicht : 398 g
Trockengehalt : 61,6 %
Asche 750 °C : 10,3 %

Ausbeute : 51,3 % = 204,2 g = 85,4 % der Theorie bezogen auf β-Naphthol.

Beispiel 2

144 g (1 Mol) β-Naphthol werden in 60 g (1 Mol) Essigsäure und 100 ml Methylpyrrolidon gelöst und auf 2 bis 5 °C abgekühlt. Zu dieser Lösung werden 345 ml $NaNO_2$ 20 Vol.-%ig (1 Mol) während 6 Stunden eindosiert. Die Nitrosierung wird polarographisch verfolgt. Die Umsetzung bei der Nitrosierung erfolgt quantitativ. Der pH-Wert liegt nach Beendigung der Reaktion bei 5,8. Innerhalb von 20 Minuten läßt man dann 532 g $NaHSO_3$ 40 %ig (2,05 Mol) zulaufen. Nach Zugabe sollte der pH-Wert nicht unter 5,5 liegen, gegebenenfalls wird mit NaOH nachgestellt. Es wird über Nacht bei Raumtemperatur gerührt bzw. solange, bis bei der polarographischen Kontrolle der Sulfit-Gehalt unter 0,1 % liegt.

Am nächsten Morgen gibt man in 2 Stunden 142,6 g $H_2SO_4$ 96 %ig zu. Die Temperatur steigt langsam an und sollte bei 60 % gehalten werden. Nach ca. 5 bis 6 Stunden wird auf 10 °C abgekühlt und abgepreßt. Der Preßkuchen wird mit 500 ml $H_2O$ neutral gewaschen.

Ausbeute : ca. 85 % bezogen auf β-Naphthol.

**Patentansprüche**

1. Verfahren zur Herstellung von 1-Amino-2-naphthol-4-sulfonsäure durch Nitrosierung von β-Naphthol, Addition von Bisulfit und Sauerstellen mit Mineralsäure, dadurch gekennzeichnet, daß man in Wasser in Gegenwart von wassermischbaren ein- oder mehrwertigen Alkoholen, deren Ethern oder Estern, cyclischer Ether, Sulfone, Amide, Nitrile, Lactame, Lactone, Sulfolan oder Sulfolen oder deren in α- oder β-Stellung durch Alkyl oder Hydroxyalkyl substituierten Derivaten, Dimethylsulfoxid, Dioxan oder Glykolformal und einer Säure mit einem Nitrit nitrosiert und anschließend mit Bisulfit umsetzt.

2. Verfahren nach Anspruch 1, dadurch gekennzeichnet, daß man 5 bis 50 Gew.-% organische Lösungsmittel verwendet.

3. Verfahren nach Ansprüchen 1 und 2, dadurch gekennzeichnet, daß man 7 bis 20 Gew.-% organische Lösungsmittel verwendet.

4. Verfahren nach Ansprüchen 1 bis 3, dadurch gekennzeichnet, daß man $C_1$-$C_4$-Alkohole, Pyrrolidon oder N-Methylpyrrolidon verwendet.

**Claims**

1. Process for the preparation of 1-amino-2-naphthol-4-sulphonic acid by the nitrosation of β-naphthol, the addition of a bisulphite and acidification using a mineral acid, characterised in that nitrosation is carried out with a nitrite in water in the presence of water-miscible mono- or polyhydric alcohols, their ethers or esters, cyclic ethers, sulphones, amides, nitriles, lactams, lactones,

sulpholane or sulpholene or their derivatives which are substituted in the α- or β-position by alkyl or hydroxyalkyl, dimethyl sulphoxide, dioxane or glycol formal and an acid and the resulting mixture is then reacted with a bisulphite.

2. Process according to Claim 1, characterised in that 5 to 50 % by weight of organic solvents are used.

3. Process according to Claims 1 and 2, characterised in that 7 to 20 % by weight of organic solvents are used.

4. Process according to Claims 1 to 3, characterised in that $C_1$-$C_4$ alcohols, pyrrolidone or N-methylpyrrolidone are used.

**Revendications**

1. Procédé de production d'acide 1-amino-2-naphtol-4-sulfonique par nitrosation de β-naphtol, addition de bisulfite et acidification avec un acide minéral, caractérisé en ce qu'on effectue la nitrosation avec un nitrite dans l'eau en présence d'alcools monovalents ou polyvalents, de leurs éthers ou esters miscibles à l'eau, d'éthers cycliques, de sulfones, d'amides, de nitriles, de lactames, de lactones, de sulfolane ou de sulfolène ou de leurs dérivés substitués en positions α ou β par des restes alkyle ou hydroxyalkyle, de diméthylsulfoxyde, de dioxanne ou de formal du glycol et d'un acide, puis on le fait réagir avec un bisulfite.

2. Procédé suivant la revendication 1, caractérisé en ce qu'on utilise 5 à 50 % en poids de solvants organiques.

3. Procédé suivant les revendications 1 et 2, caractérisé en ce qu'on utilise 7 à 20 % en poids de solvants organiques.

4. Procédé suivant les revendications 1 à 3, caractérisé en ce qu'on utilise des alcools en $C_1$ à $C_4$, de la pyrrolidone ou de la N-méthylpyrrolidone.